# EUROPEAN PATENT APPLICATION

(11) **EP 1 207 384 A2**
(43) Date of publication of application: **22.05.2002**
(21) Application number: 01660192.4
(22) Date of filing: 15.10.2001
(51) Int. Cl.: G01N 19/10

(54) **Method and device for determination of the water content in a granular material**

(30) Priority: 16.10.2000 FI 20002280
(71) Applicant: Addtek Research & Development Oy Ab, 01510 Vantaa (FI)
(72) Inventor: Käppi,Aulis, 21600 Parainen (FI); Nordenswan, Erik, 21600 Parainen (FI); Kuosmanen, Olavi, 21600 Parainen (FI); Skyttä, Olli, 20540 Turku (FI); Viljamaa, Mikko, 20810 Turku (FI)
(74) Representative: Langenskiöld, Tord Karl Walter

(57) **Abstract**

A method and device for measuring the moisture content and workability of a granular material. According to the method, use is made of the fact that the bulk density of a granular material, like sand, initially decreases to a minimum with rising moisture content and subsequently increases. When the volume and weight of a material mixture is measured, the moisture content can be determined using a known curve, and thus for example the amount of water can be correctly adjusted in the production of concrete. Thus, the desired workability is obtained. Preferably, the volume is determined by laser measurement of the surface level of concrete being mixed.

## Description

This invention relates to a method and device for determining the moisture content and workability of a granular material or a mixture containing a granular material.

It is known, that water is the dominant factor affecting the workability of concrete. Workability is often expressed as plasticity or compressibility. It is also known, that the volume of a granular material in a loose state or compressed by a standard method, is dependant on its moisture content. In this context, a granular material means a material composed of solid particles of similar or various sizes. The phenomenon is known as bulking. The meniscuses appearing between particles due to water films and other surface forces resist close packing at moisture content values between given limits, reaching a maximum at a certain moisture content between the extremes. When the moisture content rises sufficiently, the separate water films merge, the plasticity of the mass rises and compression occurs more easily. The phenomenon is illustrated by that completely dry sand, by mere gravity, occupies a smaller volume than moist sand. As sufficient water is added to dry sand, the sand becomes plastic and when saturated it occupies the same volume as completely dry sand, without compression. A moist mixture of solid material is produced, the volume of which changes as water is added.

When hollow-core concrete slabs are manufactured by continuous casting, the mass shall be relatively stiff to prevent the slab from collapsing at the cavities. Therefore it is important, that the casting mass has the right water content and thus the workability depending thereon. However, the moisture content of the sand used for the mass varies according to prevailing conditions, and thus the amount of water added to the mass along with the sand is not constant, and in practice it cannot be determined with sufficient accuracy using known methods.

In factory conditions, the compressibility of stiff concrete mass has been evaluated by squeezing concrete mass in the hand and, relying on experience, assessing the suitability of the relevant mass for, e.g. hollow-core slab production on a scale of too dry - proper - wet. Also visual evaluation has been done during mixing of mass.

Mainly for laboratory conditions, a rotational compression device has been developed, for measuring the density of a concrete sample immediately after a certain compression work has been applied. The workability (and moisture content) of a granular material can be measured by measuring the density of a sample of known mass after a standard compression. A higher achieved density reflects better workability and higher moisture content.

The weakness of the aforementioned manual and visual evaluation methods is inaccuracy. From the standpoint of factory production control, the drawback of a measurement by the rotational compression method described above is that the measurement takes 3-6 minutes. This is too long a period from a process point of view. In addition, the prior art device is expensive and sensitive when used in a rough environment. From a process control point of view, the device should be able to measure compressibility quickly and directly, e.g. from a concrete mixer. No known, industrially implemented moisture measurement method is sufficiently accurate even in a conventional environment. The problems are accentuated when recycled mineral material is used, such as crushed concrete, whereby the pH and conductivity of the mineral mixture vary and disturb resistance measurement as well as microwave measurement In addition, prior art methods are susceptible to variations in granularity, temperature and material bulk density. Thus they are fairly appropriate only for limited ranges of moisture and limited material compositions.

In U.S. patent 2,719,018, a method and device are disclosed for determining the density of a liquid-solid suspension and for measuring the proportion of liquid or solid therein. The publication does not deal with determining the moisture content of such a granular material, which has an open structure of particles whereby the differences in density are caused by the proportion of surface forces or meniscuses formed by the interfaces between air and the thin liquid film on the surface of individual particles, resulting in a more or less spacious, air-containing structure.

In analogy with the above, in publication EP 0 016 613, a suspension is discussed which is not a granular material and the density variations of which are not caused by the amount of air contained in the material.

In the method disclosed in publication DE 1 112 40, the total weight of a standard volume sample container and a granular sample is measured, and compared to the weight of a corresponding sample container containing dry material. It is assumed, that the additional weight is due to water. This method does not take into account the fact, that as a container is filled using a standard quantity of work, the amount of solids it will hold is not constant if the moisture content varies. Thus, the method is not valid for sands, and it is in conflict with the generally known moisture expansion of fine granular materials.

The characteristics of the method according to the present invention are set forth in claim 1. The characteristics of a device according to the invention are set forth in claim 4.

Because the variation in density of a granular material following handling (transport, transfer, turning over or similar stirring operations) in many processes depends primarily on a change in moisture content of the material, and the bulk density of a granular material at certain moisture contents changes strongly in response to a moisture change, the moisture content can be determined on the basis of bulk density.

The most convenient manner of measuring the relevant bulk density is to weigh a known mass of granular material into a container and measure the volume of the material. If the shape of the container and the location of its bottom are known, measuring the upper surface of the material is sufficient. The measurement can be carried out e.g. by an ultrasound measuring device, or a device utilizing electromagnetic waves. The latter may be based on visible light, infrared radiation, laser or radar. The use of a mechanical probe is also possible.

It is essential for the present invention that the measurement takes place in real time during the process.

The change of bulk density due to moisture in a granular material is the more accentuated, the smaller the individual particles. The surface forces caused by water films between smaller particles relative to the particle size makes a more spacious cell-like structure possible. Thus, the addition of moisture to a material having small particles causes a greater increase in volume than in a material having large particles.

When a method according to the invention is applied to e.g. measuring the workability of stiff concrete, it is preferable to measure a mixture having as many small particles as possible, i.e. mass containing cement. The measurement can also be done on a fraction of mere sand, because it is generally the main source of moisture variations. In some cases it is advantageous to add a known amount of water to the mass to be measured, in order to reach a range on the volume-change curve that is appropriate for measurement.

According to this invention, the volume measurement of the sample preferably takes place without any particular compression work, directly in e.g. a concrete mixer, where the aforementioned surface forces affect the sample volume, in addition to gravity.

Because a known amount of concrete raw materials, the density of each being known, is measured into a concrete mixer, and as the dimensions of the concrete mixer are known, only information on the location of the top surface of the concrete mass is required for determining the bulk density. From the bulk density, moisture content is derived from a known material-specific moisture-bulk density curve.

In a mixer according to the invention, either the mixer or elements thereof move to generate a turbulent situation. Consequently, the mixed granular material mass, which is structurally more or less open, is not homogeneous or uniform, but contains air-filled cavities of various size, particularly behind and below the mixer elements.

The top surface level of the concrete mass can be easily and very accurately determined using electronic distance measuring apparatus, based on e.g. light or ultrasound, whereby the volume and bulk density of the concrete mass can be calculated. The influence of the variation in surface level of the concrete mass moving in the mixer can be eliminated through a great number of measurements.

For example, the moisture content of concrete masses used in hollow core slab production can be determined with an accuracy better than 3 l per m³ of concrete. More generally, when the moisture content of granular materials is measured, the accuracy depends on the particle size distribution and the moisture content.

As the amount of water accompanying, for example, the mineral material, is not in practice known, the measured amount of mineral material in the batch is not accurately known. This causes, in practice, only a minor error because the influence of liquid surface forces on the bulk density is multifold relative to solid material of the same weight at the mixture ratios relevant for e.g. hollow core slab concrete.

The compression of fresh concrete is affected by many factors apart from moisture, but as the same products are continuously used in the same manner at each concrete station, the influence of other factors can be neglected as irrelevant. The method according to the invention can be used alone, or in combination with another, for example a moisture measurement method based on, e.g., power output, torsional moment, conductivity, microwave suppression or phase shift.

The invention and the particulars thereof are disclosed in more detail in the following with reference to the accompanying drawings, where
Figure 1 is a schematic illustration of the relation, applicable for calibration, of bulk density and moisture content,
Figure 2 is a schematic illustration of the relation, applicable for calibration, of surface level and moisture content,
Figure 3 shows a vertical section of a concrete mixer provided with a device according to the invention.

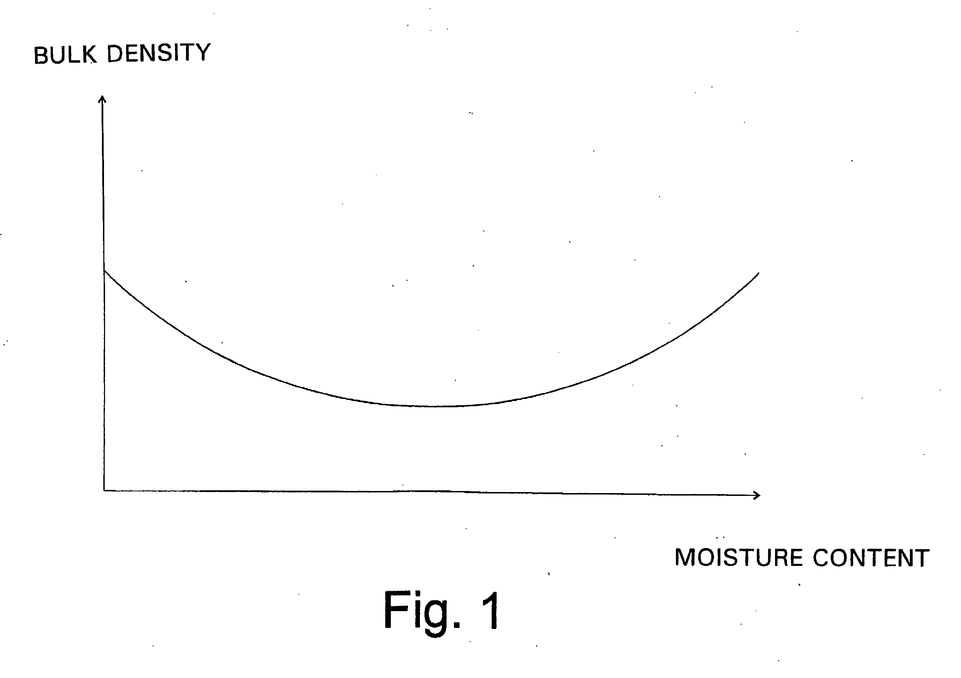

Figure 1 shows on the x-axis the percentage moisture content of a granular material such as sand, and on the y-axis schematically the bulk density thereof. What is shown is a curve depicting the moisture-bulk density relationship. It can be seen, that the bulk density firstly decreases sharply at low moisture percentages, as moisture increases, after which the curve displays a flatter minimum range. After this, the bulk density increases again. The workability of the mass, on the other hand, is related to the moisture.

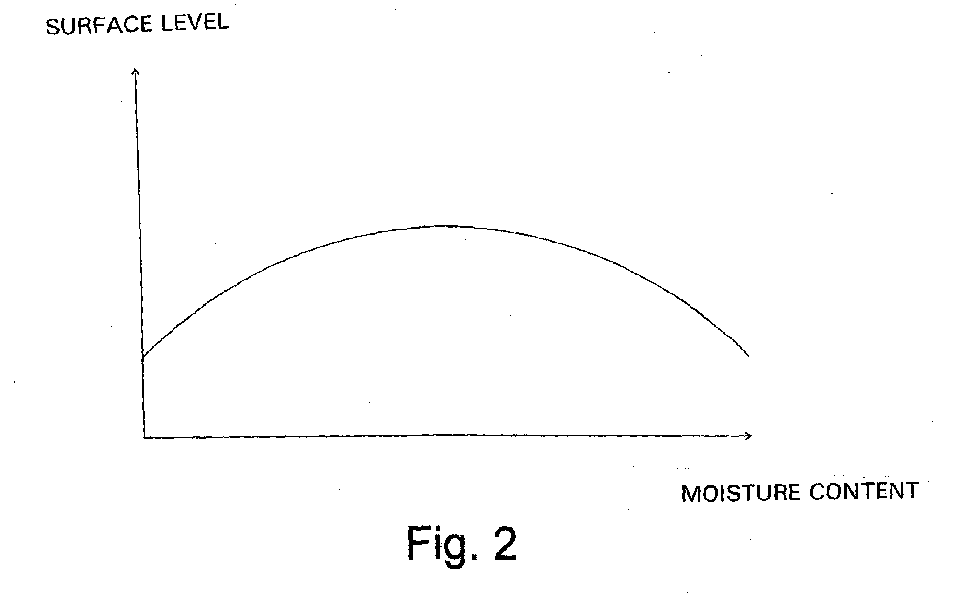

Figure 2 shows on the x-axis the percentage moisture content of a granular material such as sand, and on the y-axis schematically its surface level. What is shown is a curve depicting the moisture-surface level relationship, which is the inverse of the moisture-bulk density curve. In practice, the initial and final parts of the curve are most significant, because in these ranges the largest changes occur as moisture is added. The effects of moisture addition are difficult to observe in the flat middle range of the curve.

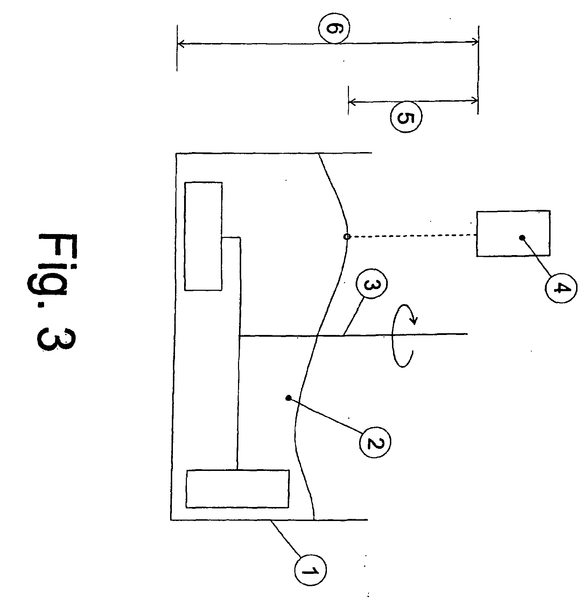

Figure 3 shows a concrete mixing container 1, mass 2 being mixed, a mixer 3 and a surface level measuring device 4. Device 4 measures the distance 5 to the mass surface. As distance 6 to the container bottom and the shape of the container are known, the volume of the container is easily determined. The mass of the contents is known from the weight metering records, and the bulk density can thus be calculated. The water content can be derived from the known moisture-bulk density curve (Fig. 1), and the amount of water to be added to the mass can be calculated.

In practice, determination of the moisture content of the mass is achieved using a moisture-surface level curve (Fig. 2). By calibrating the mass surface level to the moisture content, the applicable portion of the moisture-surface level curve is determined. The calibration is carried out for each mix and recipe defining a mixture for production and for each individual mixer by determining the mixture surface level for at least two separate moisture values, for example by means of the weight loss of a weighed and dried sample. Using this procedure, it is not necessary to know the mixture volume.

After calibration is done, the moisture content can be determined simply by measuring the mixture surface level. During production, this is carried out by charging the measured amounts of solids having an unknown moisture content to the mixer, mixing them together and measuring the surface level of the mixture. The result is compared to the moisture-surface level curve portion established by calibration; thus, the moisture content of the mixture and the required amount of additional water are determined. After water addition and mixing, the mixture is ready. After calibration, finished mixture can be produced by this procedure simply by measuring mixture surface level.

The apparatus described comprises a computing unit implemented in a manner known to the skilled person, for computing the moisture according to the moisture-bulk density relation stored in memory. The unit can naturally be part of an automation system.

### Example 1

In a metering step, the weighed concrete raw materials are charged into a mixer while it is running. The measuring unit measures the surface level of the mass consisting of mineral material, cement and possible additives, computes the required amount of additional water and adds this to the mixer.
The measuring step lasts only about 5 to 20 seconds and is thus shorter than when using prior art measuring methods.

### Example 2

Sand is metered onto a weighing unit. The volume of the sand is measured and the moisture is calculated, or the level of a selected surface point is measured, which level may be calibrated to a moisture value. From the measured value, the amount of water in the final charge is calculated, and the additional water required by the recipe is charged accordingly.

## Claims

1. A method for the determination of the moisture content and/or workability of a granular material or a mixture containing granular material, **characterised by**
a) an in-process (on-line) stage wherein
- the volume of an amount of material or mixture having a defined mass is determined, or
- the mass of an amount of material or mixture having a defined volume is determined, or
- the volume and mass of an amount of material mixture is determined
b) the determination of the required parameter on the basis of a known moisture-bulk density relation curve.

2. The method according to claim 1, **characterised by** the granular material consisting of concrete raw materials.

3. The method according to claim 1 or 2, **characterised by** the moisture of the material being determined on the basis of a moisture-surface level curve defined by a given mixer and mixture by measuring the surface level of the material or mixture.

4. A device for the determination of the moisture content and/or workability of a granular material or a mixture containing granular material, **characterised by** the device having means for mixing or transferring the material or mixture and
- means for determining the volume of an amount of material or mixture having a defined mass
- means for determining the mass of an amount of material or mixture having a defined volume
- means for determining the moisture content and/or workability based on a known moisture-bulk density relation.

5. A device according to claim 4, **characterised by** having a handling container and means for determining the surface level of the material or mixture in the container.

6. A device according to claim 4 or 5, **characterised by** the means for determining the surface level of the material or mixture being a distance measuring device based on ultrasound or electromagnetic waves, or a mechanical probe.
